## Europäisches Patentamt

## European Patent Office

## Office européen des brevets

(11) Veröffentlichungsnummer : **0 056 439**
**B1**

(12) **EUROPÄISCHE PATENTSCHRIFT**

(45) Veröffentlichungstag der Patentschrift :
**11.04.84**

(21) Anmeldenummer : **81108838.4**

(22) Anmeldetag : **24.10.81**

(51) Int. Cl.³ : **C 07 C   1/24**, C 07 C 15/073,
C 07 C 15/08, B 01 J 21/04,
B 01 J 21/06, B 01 J 23/20,
B 01 J 23/24, B 01 J 23/40,
B 01 J 23/74

(54) **Verfahren zur Herstellung von o-Xylol und Ethylbenzol.**

(30) Priorität : **15.01.81 DE 3101043**

(43) Veröffentlichungstag der Anmeldung :
**28.07.82 Patentblatt 82/30**

(45) Bekanntmachung des Hinweises auf die Patenterteilung : **11.04.84 Patentblatt 84/15**

(84) Benannte Vertragsstaaten :
**AT BE DE FR GB IT NL SE**

(56) Entgegenhaltungen :
**DE-C-   885 698**
**US-A- 4 234 752**
**Chemical Abstracts Band 70, Nr. 15, 1969 Columbus, Ohio, USA M. KRAUS "Dehydration of substituted 1-phenylethanols on oxide catalysts" Seite 283, Spalte 2, Abstract Nr. 67457t**
**Chemical Abstracts Band 69, Nr. 15, 1968 Columbus, Ohio, USA C.W. SPANGLER et al. "The catalytic dehydration of isomeric octadienols" Seite 5481, Spalte 2, Abstract Nr. 58770s**
**Chemical Abstracts Band 51, Nr. 6, 1957 Columbus, Ohio, USA G.F. WOODS et al. "Dimethyl-1,3,5-hexatrienes" Spalten 4290i bis 4292b**

(73) Patentinhaber : **BASF Aktiengesellschaft**
**Carl-Bosch-Strasse 38**
**D-6700 Ludwigshafen (DE)**

(72) Erfinder : **Weltz, Hans-Martin, Dr.**
**Auf dem Koeppel 40**
**D-6702 Bad Duerkheim (DE)**
Erfinder : **Fischer, Rolf, Dr.**
**Bergstrasse 98**
**D-6900 Heidelberg (DE)**
Erfinder : **Pohl, Hans Henning, Dr.**
**Berliner Strasse 3**
**D-6705 Deidesheim (DE)**

## Verfahren zur Herstellung von o-Xylol und Ethylbenzol

Die vorliegende Erfindung betrifft ein Verfahren zur Herstellung von o-Xylol und Ethylbenzol durch Umsetzung von 2,7-Octadien-1-ol oder 1,7-Octadien-3-ol oder von Carbonsäureestern dieser Octadienole an einem Katalysator bei Temperaturen von 200 bis 550 °C.

Es ist bekannt (G. F. Woods, A. Viola, J. Amer. Chem. Soc. 78, S. 4380 bis 4383, (1956)), daß 2,4-Octadien-6-ol bei 300 bis 310 °C an Aluminiumoxid zu 1,6-Dimethyl-1,3,5-hexatrien oder 1-Ethyl-1,3,5-hexatrien dehydratisiert wird. Führt man die Umsetzung statt bei 300 bis 310 °C bei 450 bis 500 °C durch, so erhält man substituierte Cyclohexadiene, die erst nach Dehydrierung an Palladium enthaltender Aktivkohle in Gemische aus o-Xylol und Ethylbenzol übergehen.

Es wurde nun ein vorteilhaftes Verfahren zur Herstellung von o-Xylol und Ethylbenzol gefunden, welches dadurch gekennzeichnet ist, daß man 2,7-Octadien-1-ol oder 1,7-Octadien-3-ol oder diese Octadienole enthaltende Gemische bzw. Carbonsäureester des 2,7-Octadien-1-ols oder des 1,7-Octadien-3-ols oder diese Carbonsäureester enthaltende Gemische bei Temperaturen von 200 bis 550 °C in Gegenwart von Aluminiumoxid oder Titandioxid als Katalysator oder Aluminiumoxid oder Titandioxid enthaltenden Katalysatoren umsetzt.

Es ist ein Vorteil des Verfahrens der vorliegenden Erfindung, daß o-Xylol und Ethylbenzol in einer einstufigen Reaktion aus den eingesetzten Octadienolen erhalten werden können. Ein weiterer Vorteil des neuen Verfahrens besteht darin, daß als Hauptprodukt neben wenig o-Xylol das wertvollere Ethylbenzol erhalten wird. Die bevorzugte Bildung von Ethylbenzol gegenüber der Bildung von o-Xylol war überraschend, da angenommen werden kann, daß bei der erfindungsgemäßen Umsetzung zunächst eine Dehydratisierung bzw. Säureabspaltung zum Octatrien und danach eine Dehydrocyclisierung des Octatriens zu o-Xylol und Ethylbenzol erfolgt. Aus der japanischen Patentanmeldung 46 173 (1966) ist jedoch bekannt, daß bei der Dehydrocyclisierung von 1,3,6-Octatrien neben Ethylbenzol als Hauptprodukt o-Xylol entsteht.

Die für das Verfahren der Erfindung als Ausgangsstoff zu verwendenden Octadienole werden beispielsweise durch Dimerisierung von Butadien oder von im sogenannten $C_4$-Crackschnitt enthaltenem Butadien in Gegenwart von Wasser, Kohlendioxid und Palladium-Komplexen erhalten. z. B. gemäß dem Verfahren der DE-OS 2 018 054. Als Ausgangsstoffe für das erfindungsgemäße Verfahren können 2,7-Octadien-1-ol oder 1,7-Octadien-3-ol getrennt oder Gemische dieser Octadienole verwendet werden. Entsprechend können bei Verwendung von Carbonsäureestern des 2,7-Octadien-1-ols oder 1,7-Octadien-3-ols die Carbonsäureester getrennt oder als Gemische verwendet werden. Bei Verwendung von Carbonsäureestern des 2,7-Octadien-1-ols oder 1,7-Octadien-3-ols oder diese Carbonsäureester enthaltenden Gemischen als Ausgangsstoffe kommen beispielsweise solche Carbonsäureester in Betracht, die sich von aromatischen Carbonsäuren, z. B. Benzoesäure, und vorzugsweise von niederen aliphatischen Carbonsäuren, insbesondere von niederen aliphatischen Monocarbonsäuren mit im allgemeinen 1 bis 4, vorzugsweise 1 bis 3 Kohlenstoffatomen, ableiten wie Ameisensäure, Essigsäure, Propionsäure.

Die Umsetzung gemäß der Erfindung, d. h. die Dehydratisierung und Dehydrocyclisierung in einer Stufe, wird in Gegenwart von Aluminiumoxid oder Titandioxid als Katalysator oder Aluminiumoxid oder Titandioxid enthaltenden Katalysatoren durchgeführt. Bei der Verwendung von Aluminiumoxid kann es vorteilhaft sein, Aluminiumoxidkatalysatoren zu verwenden, die zusätzlich Oxide von Elementen der Gruppen 5b, 6b und/oder 8 des Periodischen Systems und/oder Metalle der Gruppe 8 des Periodischen Systems enthalten (Periodisches System nach Handbook of Chemistry and Physics, 49. Aufl., 1968 bis 1969).

Als Oxide, die dem Aluminiumoxid zugesetzt werden können, kommen z. B. Vanadiumoxid wie $V_2O_5$, Vanadate, z. B. Alkalivanadate, Chromoxide wie $Cr_2O_3$, Chromate wie Alkalichromate, Molybdänoxid wie $MoO_3$, Molybdate wie Alkalimolybdate, Cobaltoxide wie $Co_2O_3$, Nickeloxide wie NiO in Betracht. Geeignete Metalle der Gruppe 8 des Periodischen Systems sind z. B. Palladium oder vorzugsweise Platin. Die Oxide werden dem Aluminiumoxid im allgemeinen in Mengen von 0,1 bis 50 Gew.-%, vorzugsweise 0,2 bis 20 Gew.-%, insbesondere von 0,5 bis 10 Gew.-%, bezogen auf das Aluminiumoxid, zugesetzt. Falls die Aluminiumoxid-Katalysatoren Metalle enthalten, so beträgt der Metallgehalt im allgemeinen 0,01 bis 30 Gew.-%, vorzugsweise 0,02 bis 20 Gew.-%, insbesondere 0,05 bis 5 Gew.-%, bezogen auf das Aluminiumoxid.

Die erfindungsgemäße Umsetzung wird bei Temperaturen von 200 bis 550 °C, vorzugsweise 200 bis 520 °C, insbesondere 230 bis 500 °C durchgeführt. Im allgemeinen wird die Umsetzung bei atmosphärischem Druck oder bei erhöhtem Druck, z. B. bei Drucken von 1,05 bis 30 bar durchgeführt. Es ist jedoch auch möglich, für die Umsetzung leicht verminderten Druck anzuwenden.

Es kann zweckmäßig sein, die erfindungsgemäß einzusetzenden Octadienole bzw. deren Carbonsäureester in Verdünnung mit Wasserstoff oder einem inerten Gas wie Stickstoff, Kohlendioxid, Wasserdampf oder gasförmigen oder verdampfbaren gesättigten und/oder einfach oder mehrfach olefinisch ungesättigten Kohlenwasserstoffen, z. B. Kohlenwasserstoffen mit 1 bis 8, vorzugsweise 1 bis 6 Kohlenstoffatomen umzusetzen. Das Gewichtsverhältnis von Verdünnungsmittel zu den erfindungsgemäß einzusetzenden Octadienolen beträgt im allgemeinen 1 : 100 bis 100, vorzugsweise 1 : 50 bis 50.

Im allgemeinen wird das erfindungsgemäße Verfahren in der Weise durchgeführt, daß der verdampfte erfindungsgemäß zu verwendende Ausgangsstoff, ggf. in Verdünnung mit dem Verdünnungsmittel, bei der entsprechenden Reaktionstemperatur über den Katalysator geleitet wird. Im allgemeinen betragen die Verweilzeiten am Katalysator 0,1 bis 1 000 sec., vorzugsweise 1 bis 500 sec., insbesondere 5 bis 100 sec. Aus dem Ethylbenzol und o-Xylol enthaltenden Reaktionsprodukt wird zweckmäßig o-Xylol und Ethylbenzol abgetrennt, wobei die Auftrennung vorzugsweise durch fraktionierte Destillation erfolgt. Nicht umgesetzte Ausgangsstoffe oder nicht vollständig zu den entsprechenden Aromaten umgesetzte, auf der Stufe von Zwischenprodukten, z. B. den Octatrienen, Alkylcyclohexenen oder Alkylcyclohexadienen stehengebliebene Reaktionsprodukte können zurückgeführt und erneut über den Katalysator geleitet werden.

Das nach dem erfindungsgemäßen Verfahren erhältliche Ethylbenzol ist ein wichtiges Ausgangsprodukt für die Herstellung von Styrol. o-Xylol ist ein wichtiges Ausgangsprodukt, z. B. für die Herstellung von Phthalsäureanhydrid.

Die folgenden Beispiele veranschaulichen die Erfindung.

Beispiele 1 bis 4 und Vergleichsbeispiele 1 bis 3

Die in der Tabelle angegebenen g-Mengen an Octadienolen bzw. Octatrienen läßt man pro Stunde aus einem Tropftrichter in einen auf 200 °C erhitzten Verdampfer tropfen. Das verdampfte Octadienol bzw. Octatrien wird in einem Stickstoffstrom von 4 Nl/h Stickstoff von oben nach unten durch einen Reaktor geleitet, der aus einem senkrecht stehenden, 30 cm langen Quarzrohr mit einem Durchmesser von 3,5 cm, das 200 ml Katalysator enthält, besteht. Der Reaktor wird durch Außenheizung auf die Reaktionstemperatur gebracht, die mit einem Thermoelement in der Reaktorfüllung etwa 20 cm vom oberen Ofenrand gemessen wird. Das Reaktionsgemisch wird in einem eisgekühlten Auffangefäß kondensiert und anschließend gaschromatographisch analysiert. Die Versuchsbedingungen und Versuchsergebnisse sind in der Tabelle angegeben.

(Siehe Tabelle Seite 4 f.)

Tabelle

| Beispiel Nr. | Eingesetztes Octadienol bzw. Octatrien (g) | Kataly-sator Gew.-% | Tempe-ratur °C | Roh-produkt g | Unumgesetzte Ausgangs- und Zwischenpro-dukte | Benzol | Toluol | o-Xylol | m-Xylol | p-Xylol | Ethyl-benzol | Restl. Verbin-dungen | Verhältnis von Ethylbenzol : o-Xylol |
|---|---|---|---|---|---|---|---|---|---|---|---|---|---|
| 1 | 2,7-Octadien-1-ol + 1,7-Octadien--3-ol (Molverhältnis 7 : 1) (63) | $Co_2O_3$ (5,0) $H_2MoO_4$ (13,5) $Al_2O_3$ | 400 | 40,5 | 5,9 | 0,9 | 2,7 | 26,3 | 4,2 | 1,2 | 39,8 | 19,0 | 1 : 0,66 |
| 2 | 2,7-Octadien-1-ol + 1,7-Octadien--3-ol (Molverhältnis 5 : 1 in Beispiel 2) (Molverhältnis 8 : 1 in Beispiel 3) (57) | NiO (3,0) $H_2MoO_4$ (15) $H_3PO_4$ (6) | 400 | 34 | 8,1 | 0,8 | 2,1 | 23,6 | 4,3 | 1,2 | 39,1 | 20,8 | 1 : 0,60 |
| 3 | | $Al_2O_3$ | 300 | 27,8 | 30,3 | 0,2 | 0,4 | 9,0 | 0,7 | 0,4 | 29,1 | 29,9 | 1 : 0,31 |
| 4 | 2,7-Octadien-1-ol (60) | $Co_2O_3$ (5,0) $H_2MoO_4$ (13,5) $Al_2O_3$ | 400 | 40 | 13,6 | 0,5 | 2,0 | 21,1 | 1,8 | 0,5 | 35,5 | 25,0 | 1 : 0,60 |
| Vergleichs-beispiel 1 | | $Al_2O_3$ | 300 | 45,3 | 7,0 | 1,2 | 5,9 | 28,9 | 4,4 | 1,6 | 23,8 | 27,8 | 1 : 1,21 |
| Vergleichs-beispiel 2 | 1,3,7-Octatrien (54) | $Co_2O_3$ (5,0) $H_2MoO_4$ (13,5) $Al_2O_3$ | 400 | 47,4 | 13,6 | 1,5 | 2,9 | 39,1 | 1,5 | 0,6 | 31,1 | 9,7 | 1 : 1,26 |
| Vergleichs-beispiel 3 | 2,4-Octadien--6-ol (52) | NiO (3,0) $H_2MoO_4$ (15) $H_3PO_4$ (6) $Al_2O_3$ | 400 | 34 | 7,2 | 0,3 | 0,9 | 45,7 | 2,2 | 0,4 | 27,8 | 15,5 | 1 : 1,64 |

0 056 439

## Ansprüche

1. Verfahren zur Herstellung von o-Xylol und Ethylbenzol, dadurch gekennzeichnet, daß man 2,7-Octadien-1-ol oder 1,7-Octadien-3-ol oder diese Octadienole enthaltende Gemische bzw. Carbonsäureester des 2,7-Octadien-1-ols oder des 1,7-Octadien-3-ols oder diese Carbonsäureester enthaltende Gemische bei Temperaturen von 200 bis 550 °C in Gegenwart von Aluminiumoxid oder Titandioxid als Katalysator oder Aluminiumoxid oder Titandioxid enthaltenden Katalysatoren umsetzt.

2. Verfahren nach Anspruch 1, dadurch gekennzeichnet, daß das Aluminiumoxid Oxide von Elementen der Gruppen 5b, 6b und/oder 8 des Periodischen Systems enthält.

3. Verfahren nach Anspruch 1 und 2, dadurch gekennzeichnet, daß das Aluminiumoxid Metalle der Gruppe 8 des Periodischen Systems enthält.

## Claims

1. A process for the preparation of o-xylene and ethylbenzene, wherein 2.7-octadien-1-ol, 1.7-octadien-3-ol or mixtures containing these octadienols, or carboxylates of 2.7-octadien-1-ol or of 1.7-octadien-3-ol, or mixtures containing these carboxylates, are converted at from 200 to 550 °C over aluminum oxide or titanium dioxide as catalyst, or over a catalyst containing aluminum oxide or titanium dioxide.

2. A process as claimed in claim 1, wherein the aluminum oxide contains oxides of elements of groups 5b, 6b and/or 8 of the periodic table.

3. A process as claimed in claim 1 or 2, wherein the aluminum oxide contains metals of group 8 of the periodic table.

## Revendications

1. Procédé de préparation d'o-xylène et d'éthylbenzène, caractérisé en ce que l'on fait réagir du 2,7-octadiène-1-ol ou du 1,7-octadiène-3-ol ou des mélanges contenant ces octadiénols ou un ester d'acide carboxylique du 2,7-octadiène-1-ol ou du 1,7-octadiène-3-ol ou des mélanges contenant de tels esters d'acide carboxylique, à des températures de 200 à 550 °C en présence d'oxyde d'aluminium ou de bioxyde de titane comme catalyseur ou en présence de catalyseurs contenant de l'oxyde d'aluminium ou du bioxyde de titane.

2. Procédé suivant la revendication 1, caractérisé en ce que l'oxyde d'aluminium contient des oxydes d'éléments des groupes 5b, 6b et(ou) 8 du Système Périodique.

3. Procédé suivant la revendication 1 et la revendication 2, caractérisé en ce que l'oxyde d'aluminium contient des métaux du groupe 8 du Système Périodique.